# FASCICULE DE BREVET EUROPEEN

(11) **EP 2 605 906 B1**
(45) Date de publication et mention de la délivrance du brevet: **05.04.2023**
(21) Numéro de dépôt: 11755408.9
(22) Date de dépôt: 10.08.2011
(51) Int. Cl.: B32B 27/00, C12M 1/00

(54) **DISPOSITIF BIOPHARMACEUTIQUE À USAGE UNIQUE D'ÉLABORATION, STOCKAGE, TRANSPORT D'UN PRODUIT BIOPHARMACEUTIQUE ET TUBE MULTICOUCHE CORRESPONDANT**
PHARMAZEUTISCHE VORRICHTUNG ZUR EINMALIGEN VERWENDUNG FÜR DIE AUFBEWAHRUNG UND DEN TRANSPORT EINES BIOPHARMAZEUTISCHEN STOFFES SOWIE ZUGEHÖRIGES MEHRSCHICHTIGER SCHLAUCH
SINGLE-USE BIOPHARMACEUTICAL DEVICE FOR PRODUCING, STORING, AND TRANSPORTING A BIOPHARMACEUTICAL MATERIAL, AND CORRESPONDING MULTILAYER TUBE

(30) Priorité: 17.08.2010 FR 1056637
(43) Date de publication de la demande: 26.06.2013
(73) Titulaire: SARTORIUS STEDIM FMT SAS, 13400 Aubagne (FR)
(72) Inventeur: BARBAROUX, Magali, 13112 La Destrousse (FR); GUENERON, Maréva, 13390 Auriol (FR)
(74) Mandataire: Vigand, Philippe
(86) Numéro de dépôt international: PCT/FR2011/051896
(87) Numéro de publication internationale: WO 2012/022906

(56) Documents cités:
- EP-A2- 0 136 848
- WO-A1-00/04131
- WO-A1-00/13896
- WO-A1-95/04652
- WO-A1-99/61083

## Description

L'invention est relative aux tubes biopharmaceutiques spécialement adaptés et destinés aux dispositifs biopharmaceutiques à usage unique d'élaboration, stockage, transport de produits biopharmaceutiques.

Elle vise plus spécialement un dispositif biopharmaceutique à usage unique d'élaboration, stockage, transport de produit biopharmaceutique comprenant au moins un tronçon de tube biopharmaceutique multicouche, un tube multicouche spécialement adapté et destiné à un tel dispositif et un procédé de fabrication d'un tel dispositif biopharmaceutique.

On entend ici par produit biopharmaceutique, un produit issu de la biotechnologie - milieu de culture, culture cellulaire, solution tampon... - ou un produit pharmaceutique.

On connaît des dispositifs biopharmaceutiques destinés à l'élaboration, au stockage et au transport d'un tel produit biopharmaceutique, du type comprenant au moins un - et possiblement plusieurs - récipients biopharmaceutiques, ayant un - et le plus souvent plusieurs - accès (ou ports) d'entrée ou de sortie et au moins un - et le plus souvent plusieurs - tronçons de tubes biopharmaceutiques agencés de telle sorte qu'une partie extrême d'un tronçon de tube soit - ou puisse être - associée de façon communicante, continue, rigide et étanche, d'une manière ou d'une autre (directement ou indirectement), à un accès d'un récipient, par exemple par l'intermédiaire d'un connecteur biopharmaceutique. À un tel dispositif biopharmaceutique est également généralement associé au moins un - et le plus souvent - plusieurs dispositifs d'arrêt d'écoulement du produit biopharmaceutique. Enfin, à un tel dispositif biopharmaceutique peuvent être associés, le cas échéant, un ou plusieurs systèmes biopharmaceutiques de mélange, d'aération, lesquels peuvent être qualifiés de systèmes biopharmaceutiques fonctionnels. Des exemples non limitatifs de tels dispositifs biopharmaceutiques sont des dispositifs de mélange et des bioréacteurs.

On entend ici par « tube » un objet creux, allongé d'une certaine longueur finie, de forme générale cylindrique ou pseudo cylindrique, ouvert à une extrémité ou aux deux extrémités, apte à être empli d'un certain contenu fluide pour son mouvement ou sa station. Un tel tube comprend une paroi latérale périphérique et un espace libre longitudinal, le cas échéant plusieurs espaces libres longitudinaux, séparés par une ou plusieurs parois. Le terme « tube » peut ici être considéré comme synonyme de « conduit », « conduite », « tuyau ». Il est entendu que le tube considéré est spécialement apte à l'emploi dans un dispositif biopharmaceutique d'élaboration, stockage, transport d'un produit biopharmaceutique, comme il vient d'être exposé, ce qui exclut un cathéter ou un dispositif analogue ou un tube de circulation extracorporelle du sang ou de passage de fluide lors d'une dialyse, d'une perfusion, ou d'une alimentation artificielle ou pour une destination et un usage analogues, qui relèvent d'un autre domaine technique.

Par synecdoque, le terme « tube » vise à la fois le cas où le tube est de grande longueur, telle que celle résultant de son processus de fabrication, et le cas où le tube est de longueur plus petite, telle que celle d'un tronçon provenant de la coupe réalisée dans une grande longueur et destiné à être incorporé au dispositif biopharmaceutique d'élaboration, stockage, transport d'un produit biopharmaceutiq ue.

Classiquement, les récipients et les tubes de tels dispositifs biopharmaceutiques d'élaboration, stockage, transport étaient rigides et réalisés en acier inoxydable, de sorte à permettre une mise en oeuvre récurrente. Comme l'enseigne l'article Stainless steel tubing in the biotechnology industry publié dans Pharmaceutical engineering 2001, vol. 21, n°5, pages 48-63, l'acier inoxydable est qualifié non seulement parce qu'il a une durée de vie importante, mais également pour sa disponibilité, sa machinabilité, ses propriétés d'être non corrosif, d'être non contaminant, de pouvoir être poli et présenter un fini lisse, d'être résistant et rigide, de pouvoir résister à la chaleur, de pouvoir subir un traitement chimique de stérilisation, et de pouvoir être aisément soudé. Avec un tel dispositif biopharmaceutique, il peut être envisagé d'associer un tube directement à un récipient ou à un autre tube, par soudure. D'autre part, un dispositif d'arrêt d'écoulement du produit biopharmaceutique se présente typiquement sous la forme d'une vanne.

Puis, on a envisagé de tels dispositifs biopharmaceutiques à usage unique, présentant une certaine souplesse et, à cette fin, réalisés en matière plastique. Un tel dispositif a notamment pour avantages une plus grande facilité d'installation, une réduction des temps morts, une utilisation optimale de la surface au sol, et une réduction très importante du risque de contamination croisée.

Un tel dispositif biopharmaceutique comprend au moins une - le cas échéant plusieurs - poches, au moins un - le cas échéant plusieurs - tronçons de tube et, le cas échéant, au moins un - et si le cas se présente plusieurs - connecteurs biopharmaceutiques. Ces éléments constitutifs, chacun à usage unique, sont associés entre eux de façon communicante, continue, rigide et étanche. Ils présentent une certaine souplesse et sont réalisés en matière plastique. Avec un tel dispositif biopharmaceutique, un tube est associé à un récipient proprement dit par l'intermédiaire d'un connecteur biopharmaceutique. Un dispositif d'arrêt d'écoulement du produit biopharmaceutique se présente alors typiquement sous la forme d'une pince (connue sous le nom de « clamp »), venant fermer la partie flexible du dispositif biopharmaceutique où elle se trouve, par aplatissement sur elle même.

S'agissant d'un tel dispositif biopharmaceutique, on connaît une poche biopharmaceutique dont les deux grandes parois sont soudées l'une à l'autre (poche 2D, D signifiant dimensions). Par exemple, le document EP-A-0136848 concerne une telle poche. On connaît aussi une poche 3D qui comporte deux parois d'extrémité et une paroi latérale pouvant être pliée à plat ou dépliée déployée, soudées entre elles, le volume pouvant atteindre 3.000 litres, voire plus. Une telle poche 3D est décrite par exemple dans le document WO00/04131 et est commercialisée par la société Sartorius sous la marque FLEXEL^{®} 3D.

Les parois de telles poches biopharmaceutiques sont essentiellement continues dans leur ensemble et comportent une face extérieure en contact avec l'environnement extérieur de la poche et une face intérieure avec laquelle est en contact le produit pharmaceutique emplissant la poche. La matière formant cette face intérieure peut, par exemple, être choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE), le polyfluorure de vinylidène (PVDF) et les matériaux équivalents, dans le cadre de la destination et l'application considérées.

Les parois de ces poches biopharmaceutiques sont choisies, notamment quant à leurs matières constitutives, leurs formes, leurs dimensions..., de sorte à être aptes d'une part à avoir une propriété de contention du contenu de la poche et de séparation physique entre celui-ci et l'extérieur de la poche et, d'autre part, à avoir des propriétés physiques, chimiques, biologiques, opératoires adaptées à la destination et à l'application considérées. De telles propriétés physiques, chimiques, biologiques, opératoires sont, à titre purement exemplatif et non limitatif, une propriété barrière à haut degré d'étanchéité aux gaz, à pouvoir mécaniquement contenir le produit biopharmaceutique se trouvant dans la poche, dont le volume peut être plus ou moins important, à éviter les interactions indésirables avec l'environnement ou le produit biopharmaceutique, à être propres, notamment au regard des particules ou sous l'angle biologique, à être non dégradables et présenter une haute résistance chimique, à permettre que l'élaboration, la conservation, le stockage, le transport et l'emploi du produit biopharmaceutique soit assuré avec sécurité, à pouvoir être soudées, à pouvoir être, aisément et sans détérioration, pliées et dépliées, à présenter des qualités au regard notamment de l'adhérence, de la souplesse, de l'opacité ou au contraire de la transparence, ces caractéristiques n'étant nullement exclusives d'autres.

Originellement, on a utilisé comme tube d'un tel dispositif biopharmaceutique à usage unique d'élaboration, stockage, transport de produit biopharmaceutique des tubes classiques en silicone ou en polychlorure de vinyle (PVC).

Les documents EP-A-0712354, EP-A-0999860, US 2009/0299260, WO 2010/051468 et WO 95/04652 enseignent toutefois que le PVC présente, à côté de nombre d'atouts (tels que notamment la flexibilité, la transparence, la capacité à encaisser sans dommage le pincement d'une pince (clamp) et le coût), nombre d'inconvénients au regard de l'emploi dans des dispositifs biopharmaceutiques à usage unique, par exemple sa dangerosité au regard de son recyclage et de son incinération et sa dégradation à la température). On a donc réalisé des tubes monocouche en d'autres matériaux tels que le LLDPE ou le polyéthylène à haute densité linéaire (HDPE), l'EVA, ou le polybutylène.

Le document WO 95/04652 décrit un tube multicouche qui comporte au moins une couche intérieure d'au moins un polymère thermoplastique sans chlore présentant une souplesse semblable ou supérieure à celle des tubes médicaux en PVC, et au moins une couche de surface extérieure d'au moins un deuxième polymère thermoplastique sans chlore, résistant et présentant un module de Young ne dépassant pas environ quinze fois le module de Young du premier polymère thermoplastique.

Le document EP-A-0999860 enseigne que l'utilisation de polyoléfines ou de composés à base de polyoléfines permet de surmonter les inconvénients du PVC, mais est source d'autres inconvénients tels que la destruction du tube lorsque celui-ci est pincé avec une pince (clamp).

L'état de la technique enseigne différentes réalisations (à savoir compositions) de tubes multicouches spécialement adaptés pour être intégrés dans des dispositifs biopharmaceutiques d'élaboration, stockage, transport à usage unique, du type exposé plus haut.

Ainsi, le document EP-A-0712354 décrit un tube multicouche comprenant une couche interne d'au moins un premier polymère thermoplastique non chloré ayant une flexibilité choisie et une couche superficielle extérieure d'au moins un deuxième polymère thermoplastique non chloré ayant une flexibilité choisie supérieure à celle du premier polymère thermoplastique.

Le document EP-A-0136848 décrit un tube d'accès multicouche co-extrudé comprenant une couche externe comprenant de l'EVA, une couche interne comprenant du PVC et entre elles une couche de liaison. Dans une extrémité d'un tel tube est emmanchée l'extrémité d'un tube membrane également en PVC, une liaison suffisamment rigide étant assurée entre elles au moyen d'une liaison à base de cyclohexanone.

Le document US 2010/0137838 décrit un tube multicouche d'accès à une poche médicale co-extrudé comprenant trois couches, à savoir une couche interne comprenant de 50% à 90% en poids d'un élastomère à base de PP, une couche intermédiaire comprenant de 45% à 55% en poids d'un élastomère à base de PP et de 45% à 55% en poids de PP et une couche externe comprenant de 20% à 55% en poids d'un élastomère à base de PP et de 45% à 80% en poids de PP.

Dans le domaine différent des tubes multicouches à usage médical, le document US 2009/0299260 décrit un tube multicouche ne comportant pas de PVC, et comprenant trois couches en matériaux choisis dans la famille comprenant le PE, le PP et leurs copolymères, leurs terpolymères et leurs mélanges, la couche intermédiaire comprenant au moins 60% de d'élastomère thermoplastique. Il est prévu que deux tels tubes sont associés au moyen d'un connecteur en polyoléfine par des soudures laser. Un tel tube multicouche est ici spécialement destiné à la circulation extracorporelle du sang et donc spécialement agencé pour pouvoir être associé à une pompe péristaltique. A contrario, un tel tube multicouche n'est pas destiné à être intégré dans un dispositif biopharmaceutique tel qu'il a été précédemment défini.

De même, le document EP-A-0765740 décrit un tube multicouche qui est exempt de PVC et comprend au moins deux couches, à savoir une première couche interne ou externe formée principalement d'un caoutchouc synthétique à base d'isoprène ou de PP et ayant notamment une densité et une dureté Shore choisies et qui soit apte à être stérilisé à la chaleur à une température ≥ 121 °C et une seconde couche externe ou interne formée principalement d'un copolymère de PE ou un caoutchouc synthétique choisi. Un tel tube multicouche est ici spécialement destiné au passage de fluide lors d'une dialyse, d'une perfusion, ou d'une alimentation artificielle. Comme précédemment, il n'est pas destiné à être intégré dans un dispositif biopharmaceutique tel qu'il a été précédemment défini.

Egalement, le document WO 99/61083 décrit un tube de pompage à une ou plusieurs couches, à usage médical, mais non pas intégré dans un dispositif biopharmaceutique tel qu'il a été précédemment défini.

Le document WO 00/13896 décrit des structures multicouches comprenant au moins une couche constituée d'un copolymère d'éthylène/alpha-oléfine ayant une densité inférieure à 0,916 g/cc et une température de fusion maximale supérieure à 118 °C. Ces structures multicouches comportent au moins trois couches. Ces structures multicouches permettent de réaliser des articles manufacturés, notamment des films, des récipients, des poches, des emballages, des tubes, et analogues.

Par conséquent, à l'instar des poches biopharmaceutiques pour les dispositifs biopharmaceutiques à usage unique d'élaboration, stockage, transport, il existe le besoin de disposer de tubes biopharmaceutiques spécialement destinés à de tels dispositifs biopharmaceutiques, qui présentent une certaine souplesse, soient réalisés en matière plastique et soient aptes notamment du fait de leurs matières constitutives, d'une part, à avoir une propriété de contention du contenu du tube et de séparation physique entre celui-ci et l'extérieur du tube et, d'autre part, à avoir des propriétés physiques, chimiques, biologiques, opératoires adaptées à la destination et à l'application considérées. De telles propriétés physiques, chimiques, biologiques, opératoires sont, à titre purement exemplatif et non limitatif, une propriété barrière à haut degré d'étanchéité aux gaz, à pouvoir être emplis du produit biopharmaceutique, à éviter les interactions indésirables avec l'environnement ou le produit biopharmaceutique, à être propres, notamment au regard des particules ou sous l'angle biologique, à être non dégradables et présenter une haute résistance chimique, à permettre que l'élaboration, la conservation, le stockage, le transport et l'emploi du produit biopharmaceutique soit assuré avec sécurité, à pouvoir être soudés, à pouvoir être courbés aisément et sans détérioration, à présenter des qualités au regard notamment de l'adhérence, de l'opacité ou au contraire de la transparence, de la souplesse, jusqu'à pouvoir être fermés par aplatissement sur lui-même au moyen d'une pince (clamp), ces caractéristiques n'étant nullement exclusives d'autres.

En outre, il existe le besoin de disposer de tels tubes biopharmaceutiques spécialement destinés à de tels dispositifs biopharmaceutiques adaptés aux exigences de plus en plus grandes de flexibilité et de polyvalence, en fonction notamment du produit biopharmaceutique concerné, de la nature et des caractéristiques du dispositif biopharmaceutiques d'élaboration, stockage, transport à usage unique mis en oeuvre et des applications réalisées à l'aide de ces dispositifs. En même temps, il convient que les utilisateurs aient toutes garanties en ce qui concerne les matériaux constitutifs de tels tubes et tout spécialement du matériau de la face intérieure du tube en contact avec le produit biopharmaceutique.

Il existe également le besoin que le - ou les - tubes biopharmaceutiques d'un tel dispositif biopharmaceutique à usage unique puissent être associés de façon communicante, continue, rigide et étanche à la - ou aux - poches biopharmaceutiques du dispositif, le cas échéant par l'intermédiaire d'un - ou de - connecteurs biopharmaceutiques, le cas échéant à un - ou des - autres tubes biopharmaceutiques. Et il est souhaitable que le matériau de la face interne de l'ensemble des éléments constitutifs du dispositif biopharmaceutique à usage unique en contact avec le produit biopharmaceutique soit le même et qu'il y ait une continuité physique parfaite, du moins la plus grande continuité physique possible entre les différentes faces intérieures successives des différents éléments constitutifs du dispositif biopharmaceutique.

Le problème à la base de l'invention est de répondre à l'ensemble de ces besoins et exigences.

La solution apportée à ce problème est selon un premier aspect un dispositif biopharmaceutique à usage unique d'élaboration, stockage, transport de produit biopharmaceutique, comprenant au moins un tronçon de tube biopharmaceutique multicouche, ayant une paroi latérale périphérique limitant un espace libre longitudinal et comportant une couche contact et une couche fonctionnelle avec une solidarisation entre la face externe de la couche contact et la face interne de la couche fonctionnelle, au moins une poche biopharmaceutique, et, le cas échéant, au moins un connecteur biopharmaceutique, chacun à usage unique et comportant une couche contact constituée d'une matière choisie apte à être au contact du produit biopharmaceutique sans dégradation de la couche contact et du produit biopharmaceutique et à être soudable sur elle-même, soudés entre eux de façon communicante, continue, rigide et étanche, présentant une certaine souplesse et réalisés en matière plastique.

Un tel dispositif biopharmaceutique est tel que :
- la couche contact du tube biopharmaceutique est constituée d'une matière choisie apte à être au contact du produit biopharmaceutique sans dégradation de la couche contact et du produit biopharmaceutique et à être soudable sur elle-même, autre que le PVC et choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE) le polyfluorure de vinylidène (PVDF), dont la face intérieure limite l'espace libre longitudinal et est apte à être au contact du produit biopharmaceutique, c'est-à-dire est apte essentiellement à assurer la fonction de contention et de séparation physique du au moins un tube biopharmaceutique et formant la face intérieure de celui-ci,
- la couche fonctionnelle du tube biopharmaceutique comprend au moins une couche élémentaire fonctionnelle constituée d'une matière choisie pour sa fonction de flexibilité, de robustesse, de manipulation, d'opacité ou au contraire de transparence, de barrière aux gaz, non dégradable à elle seule ou par association à une couche protectrice, autre que le PVC et choisie dans la famille comprenant le polyéthylène (PE) et notamment le polyéthylène à basse densité linéaire (LLDPE), le polyéthylène téréphtalate (PET), un polyamide (PA), le polyéthylène vinyle acétate (EVA), l'éthylène-alcool vinylique (EVOH), le styrène éthylène butadiène-styrène (SEBS), le polyéthylène téréphtalate glycol (PETG...), le polyfluorure de vinylidène (PVDF), c'est-à-dire apte à assurer les fonctions permettant de satisfaire les autres propriétés physiques, chimiques, biologiques, opératoires adaptées à la destination et à l'application considérées du dispositif biopharmaceutique à usage unique,
- la couche contact de la poche, d'un autre tronçon de tube et/ou d'un connecteur, est constituée d'une matière choisie identique ou analogue à la matière de la couche contact du au moins un tube biopharmaceutique,
- la couche contact du tube biopharmaceutique est soudée à la couche contact de la poche, d'un autre tronçon de tube et/ou d'un connecteur, avec une continuité physique substantielle entre les couches contact respectives.

Selon une réalisation, une poche biopharmaceutique, le cas échéant un autre tronçon de tube biopharmaceutique et/ou un connecteur biopharmaceutique, soudé au au moins un tube biopharmaceutique, a une structure multicouche identique ou analogue à la structure multicouche du au moins un tube biopharmaceutique multicouche, à savoir comprenant une couche contact et une couche fonctionnelle comprenant au moins une couche élémentaire fonctionnelle. En particulier, la couche fonctionnelle du au moins un tube biopharmaceutique est soudée à la couche fonctionnelle de la poche biopharmaceutique, le cas échéant de l'autre tronçon de tube biopharmaceutique et/ou du connecteur biopharmaceutique.

Selon les réalisations, le au moins un tube biopharmaceutique est soudé à un autre élément constitutif du dispositif biopharmaceutique soit directement formant ainsi intrinsèquement connecteur biopharmaceutique soit indirectement par l'intermédiaire d'un connecteur biopharmaceutique.

Selon une réalisation, au dispositif biopharmaceutique à usage unique d'élaboration, stockage, transport de produit biopharmaceutique est associé structurellement et fonctionnellement, de façon amovible ou inamovible, au moins un dispositif d'arrêt d'écoulement du produit biopharmaceutique dans l'espace libre longitudinal du au moins un tube biopharmaceutique ayant la forme d'une pince apte à fermer la partie du au moins un tube biopharmaceutique où elle se trouve par aplatissement du moins un tube biopharmaceutique sur lui-même.

Selon les réalisations, le dispositif biopharmaceutique à usage unique d'élaboration, stockage, transport de produit biopharmaceutique constitue une poche de stockage et/ou remplissage et/ou manipulation et/ou transport et/ou mélange d'un produit biopharmaceutique ou un bioréacteur d'un produit biopharmaceutique.

Selon une réalisation, le tube biopharmaceutique multicouche est constitué de la couche contact et de la couche fonctionnelle, la couche fonctionnelle formant la face extérieure du tube biopharmaceutique multicouche.

Selon une première réalisation, le tube biopharmaceutique multicouche comprend une unique couche élémentaire fonctionnelle. Selon une seconde réalisation, il comprend une pluralité de couches élémentaires fonctionnelles, chacune solidarisée à la ou aux couches adjacentes.

Selon une réalisation, la solidarisation entre la face externe de la couche contact et la face interne de la couche fonctionnelle est assurée de fabrication, par co extrusion ou enrobage ou gainage.

Selon un mode particulier de la première réalisation, le tube biopharmaceutique multicouche comporte, associée à une couche contact de LLDPE, une couche élémentaire fonctionnelle de PE.

Selon un mode particulier de la seconde réalisation, le tube biopharmaceutique multicouche comporte, associées à une couche contact de LLDPE, une couche élémentaire fonctionnelle d'EVOH, une couche élémentaire fonctionnelle de PA et une couche élémentaire fonctionnelle de PET qui forme la face extérieure du tube biopharmaceutique multicouche.

Selon un autre mode particulier de la seconde réalisation, le tube biopharmaceutique multicouche comporte, associées à une couche contact de LLDPE, une couche élémentaire fonctionnelle d'EVOH, une couche élémentaire fonctionnelle de PA, deux couches élémentaires fonctionnelles de LLDPE et une couche élémentaire fonctionnelle en matériau biodégradable qui forme la face extérieure du tube biopharmaceutique multicouche.

Selon un autre mode particulier de la seconde réalisation, le tube biopharmaceutique multicouche comporte, associées à une couche contact de LLDPE, une couche élémentaire fonctionnelle en matériau biodégradable, deux couches élémentaires fonctionnelles de LLDPE, une couche élémentaire fonctionnelle d'EVOH et une couche élémentaire fonctionnelle de PA qui forme la face extérieure du tube biopharmaceutique multicouche.

Selon un autre mode particulier de la seconde réalisation, le tube biopharmaceutique multicouche comporte, associées à une couche contact de PE, une couche élémentaire fonctionnelle d'EVA, une couche élémentaire fonctionnelle d'EVOH, une couche élémentaire fonctionnelle d'EVA et une couche de PE qui forme la face extérieure du tube biopharmaceutique multicouche.

Selon un autre mode particulier de la seconde réalisation, le tube biopharmaceutique multicouche comporte, associées à une couche contact de LLDPE, une couche élémentaire fonctionnelle d'EVOH, une couche élémentaire fonctionnelle de LLDPE et une couche élémentaire fonctionnelle de PE qui forme la face extérieure du tube biopharmaceutique multicouche.

Selon une réalisation, le tube biopharmaceutique multicouche est à section transversale circulaire ou oblongue.

Selon les réalisations, le tube biopharmaceutique multicouche comporte un espace libre longitudinal unique ou plusieurs espaces libres longitudinaux juxtaposés avec séparation étanche.

Selon les réalisations, le tube biopharmaceutique multicouche est homogène d'une extrémité à l'autre du tronçon qu'il forme ou, au contraire, il comporte à au moins une extrémité du tronçon qu'il forme une partie saillante découverte constituée de la couche de contact, un tel tronçon de tube biopharmaceutique multicouche faisant également fonction de connecteur pour le dispositif biopharmaceutique à usage unique d'élaboration, stockage, transport de produit biopharmaceutique.

Selon un troisième aspect, l'invention a pour objet un procédé de fabrication d'un dispositif biopharmaceutique à usage unique d'élaboration, stockage, transport de produit biopharmaceutique tel qu'il a été précédemment décrit, dans lequel on dispose d'au moins une poche biopharmaceutique, d'au moins un tronçon de tube biopharmaceutique et, le cas échéant, d'au moins un connecteur biopharmaceutique, et on positionne ces éléments dans la configuration du dispositif biopharmaceutique à fabriquer et on les soude entre eux de façon communicante, continue, rigide et étanche, de sorte que la couche contact du au moins un tube biopharmaceutique soit soudée à la couche contact de la poche biopharmaceutique, d'un autre tronçon de tube biopharmaceutique ou le cas échéant du connecteur biopharmaceutique avec une continuité physique substantielle entre les couches contact respectives.

On décrit maintenant plusieurs modes de réalisation de l'invention à l'aide des dessins, dans lesquels :

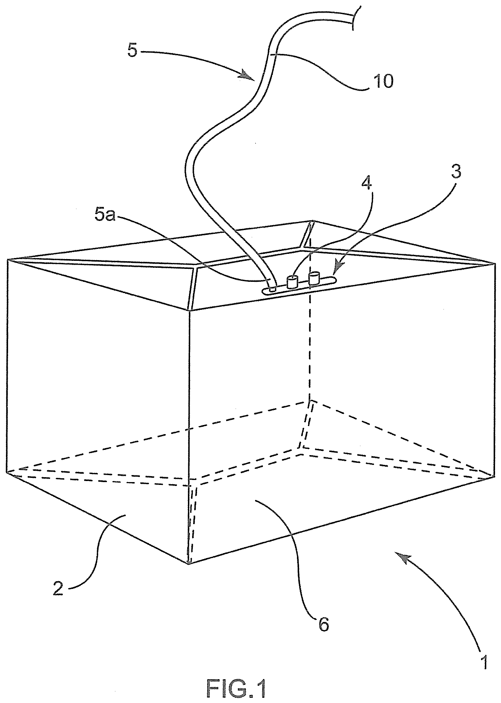

- La figure 1 est une vue en perspective d'une réalisation possible et exemplative d'un dispositif biopharmaceutique selon l'invention, à usage unique, pour l'élaboration, le stockage et le transport d'un produit biopharmaceutique, comprenant en l'espèce une poche biopharmaceutique, un connecteur biopharmaceutique et un tronçon de tube biopharmaceutique, chacun à usage unique, soudés entre eux de façon communicante, continue, rigide et étanche, présentant une certaine souplesse et réalisés en matière plastique.

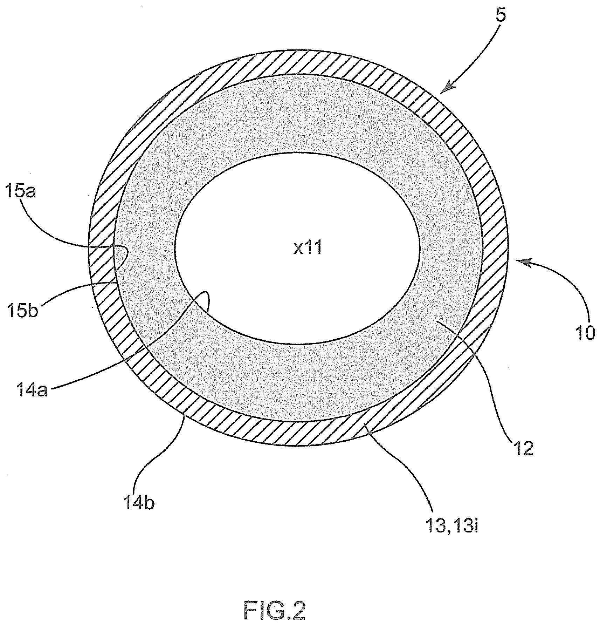

- La figure 2 est une vue en coupe transversale du tronçon de tube biopharmaceutique multicouche faisant partie du dispositif biopharmaceutique à usage unique de la figure 1, selon une réalisation possible et exemplative dans laquelle le tube comprend une couche contact et une couche élémentaire fonctionnelle.

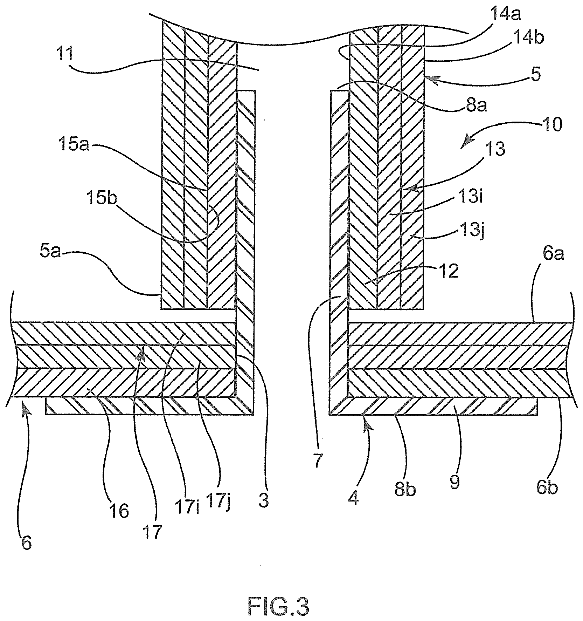

- La figure 3 est une vue partielle en coupe longitudinale transversale illustrant l'association communicante, continue, rigide et étanche de la poche biopharmaceutique, du connecteur biopharmaceutique et du tronçon de tube biopharmaceutique de la figure 1.

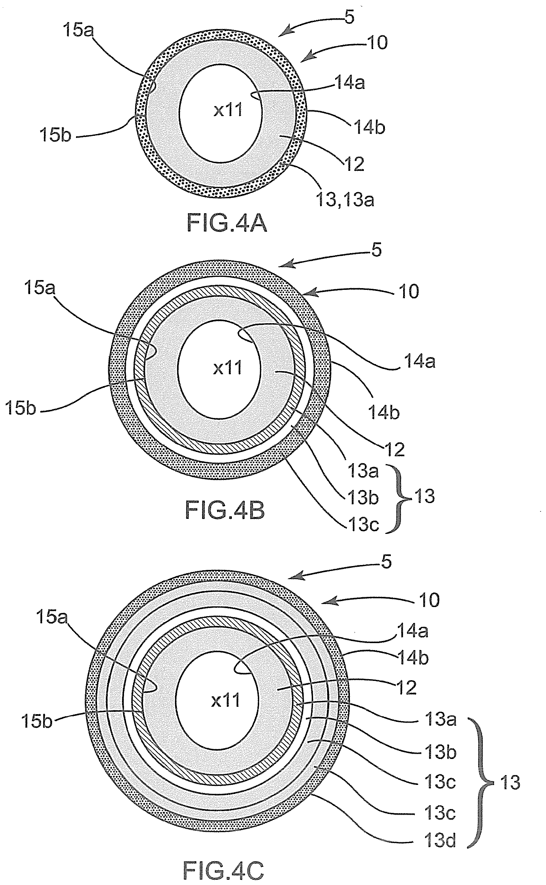

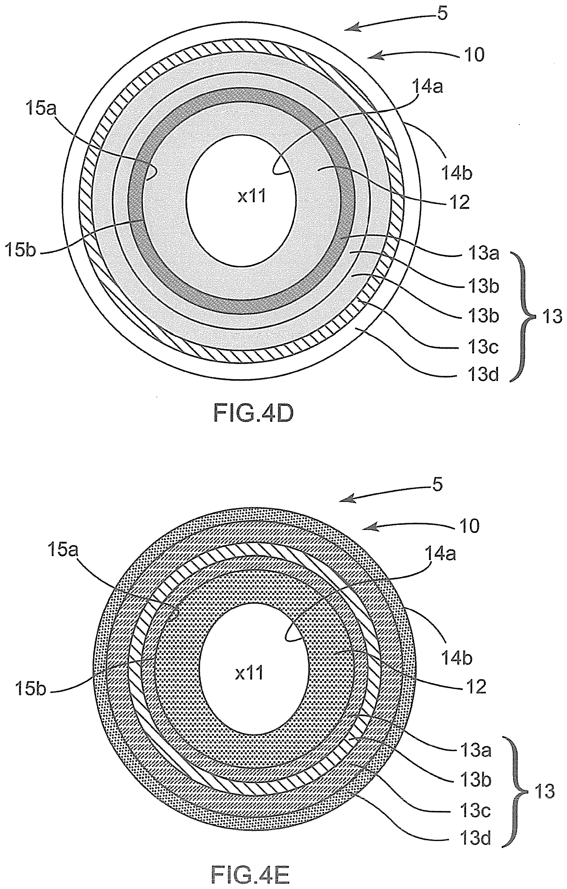

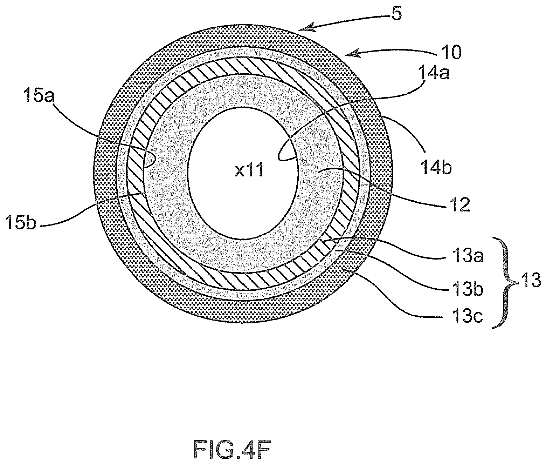

- Les figures 4A, 4B, 4C, 4D, 4E et 4F sont six vues en coupe transversale, partielles illustrant différentes réalisations de tubes biopharmaceutiques multicouches, à savoir, un tube comprenant une couche contact de LLDPE et une unique couche élémentaire fonctionnelle de PE (figure 4A), un tube comprenant une couche contact de LLDPE et des couches élémentaires fonctionnelles d'EVOH, de PA et de PET (figure 4B), un tube comprenant une couche contact de LLDPE et des couches élémentaires fonctionnelles d'EVOH, de PA, de LLDPE et en matériau biodégradable (figure 4C), un tube comprenant une couche contact de LLDPE et des couches élémentaires fonctionnelles en matériau biodégradable, en LLDPE, en EVOH, en PA (figure 4D), un tube comprenant une couche contact de PE et des couches élémentaires fonctionnelles d'EVA, d'EVOH, d'EVA et de PE (figure 4E), un tube comprenant une couche contact de LLDPE et des couches élémentaires fonctionnelles d'EVOH, de LLDPE et de PE (figure 4F).

Sur la figure 1 est représentée une réalisation possible, purement exemplative et non limitative, d'un dispositif biopharmaceutique 1, à usage unique destiné à l'élaboration, au stockage et au transport d'un produit biopharmaceutique.

On entend par produit biopharmaceutique, un produit issu de la biotechnologie - milieu de culture, culture cellulaire, solution tampon... - ou un produit pharmaceutique.

Le dispositif biopharmaceutique 1 représenté comprend un récipient biopharmaceutique, ici une poche 2, à trois dimensions (poche 3D), ayant des accès (ou ports) d'entrée ou de sortie 3, un connecteur biopharmaceutique 4 creux, et un tronçon de tube biopharmaceutique 5 ayant à l'une de ses extrémités, une partie extrême 5a. Chacun de ces éléments constitutifs 2, 4, 5 du dispositif biopharmaceutique 1 est à usage unique et réalisé en matière plastique de sorte à présenter une certaine souplesse (surtout pour la poche 2 et le tronçon de tube 5, le connecteur 4 pouvant être plus rigide le cas échéant). Dans le dispositif biopharmaceutique 1 monté, ces éléments constitutifs 2, 4, 5, sont associés entre eux fonctionnellement et structurellement en étant soudés entre eux de façon communicante, continue, rigide et étanche selon l'agencement correspondant à la destination et à l'application considérées. Le cas échéant, dans le dispositif biopharmaceutique 1 monté, un tronçon de tube biopharmaceutique 5 peut être associé fonctionnellement et structurellement à un autre tronçon de tube biopharmaceutique 5, les caractéristiques de celui-ci étant identiques ou analogues aux caractéristiques de celui-là.

Selon une autre réalisation purement exemplative et non limitative, le tronçon de tube biopharmaceutique 5 forme lui-même connecteur biopharmaceutique, étant associé directement à la poche biopharmaceutique 2, sa partie extrême comportant une collerette transversale, dirigée vers l'extérieur, analogue à la collerette transversale 9 d'un connecteur biopharmaceutique 4 décrit par la suite.

Une poche 2, 3D est décrite par exemple dans le document WO00/04131. Cette réalisation est purement exemplative et non limitative. La poche 2 du dispositif biopharmaceutique 1 peut également être de type 2D, avec deux grandes parois soudées l'une à l'autre.

Les parois 6, latérales périphériques, de telles poches biopharmaceutiques 2 sont essentiellement continues dans leur ensemble et comportent une face extérieure 6a en contact avec l'environnement extérieur de la poche 2 et une face intérieure 6b avec laquelle est en contact le produit pharmaceutique emplissant la poche.

Le terme « extérieur » se rapporte à ce qui est au dehors du dispositif 1 ou de l'élément constitutif considéré (notamment l'ambiance dans laquelle il se trouve), le terme « intérieur » se rapporte à ce qui est au-dedans du dispositif 1 ou de l'élément constitutif considéré (notamment ce qui est contact ou apte à être au contact du produit biopharmaceutique).

Le terme « externe » se rapporte à ce qui est tourné vers l'extérieur ou est situé le plus vers l'extérieur, tandis que le terme « interne » se rapporte à ce qui est tourné vers l'intérieur ou est situé le plus vers l'intérieur. Les termes « externe » et « interne » doivent donc être considérés de façon relative.

Les parois 6 de telles poches biopharmaceutiques 2 sont aptes à avoir une propriété barrière à haut degré d'étanchéité aux gaz, à pouvoir mécaniquement contenir le produit biopharmaceutique se trouvant dans la poche 2, à éviter les interactions indésirables avec l'environnement ou le produit biopharmaceutique, à être propres, notamment au regard des particules ou sous l'angle biologique, à être non dégradables et présenter une haute résistance chimique, à permettre que l'élaboration, la conservation, le stockage, le transport et l'emploi du produit biopharmaceutique soit assuré avec sécurité, à pouvoir être soudées, à pouvoir être, aisément et sans détérioration, pliées et dépliées ou gonflées et dégonflées, à présenter des qualités au regard notamment de l'adhérence, de la souplesse, de l'opacité ou au contraire de la transparence, ces caractéristiques n'étant nullement exclusives d'autres.

La matière formant la face intérieure 6b d'une telle poche biopharmaceutique 2 est, dans la réalisation considérée, choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE), le polyfluorure de vinylidène (PVDF) et les matériaux équivalents, dans le cadre de la destination et de l'application considérées. En aucun cas la matière formant la face intérieure 6b est du PVC.

Dans le dispositif biopharmaceutique 1 monté, la partie extrême 5a du tronçon de tube biopharmaceutique 5 est associée de façon communicante, continue, rigide et étanche, par soudage, à l'accès 3 par l'intermédiaire du connecteur biopharmaceutique 4, lui-même associé de façon communicante, continue, rigide et étanche, par soudage, à la paroi 6 de la poche 2 à l'endroit de l'accès 3.

Dans une réalisation purement exemplative et non limitative, le connecteur biopharmaceutique 4 qui présente un axe central, comporte, d'un seul tenant, un tronçon creux 7 de forme générale cylindrique ou pseudo cylindrique, ouvert aux deux extrémités 8a et 8b, et une collerette transversale 9, dirigée vers l'extérieur, c'est-à-dire en s'éloignant de l'axe du connecteur 4.

La collerette transversale 9 est soudée à la paroi 6 de la poche biopharmaceutique 2, autour d'un trou ménagé dans la paroi 6 et formant l'accès 3. Le tronçon creux 7 est soudé à la partie extrême 5a du tronçon de tube biopharmaceutique 5, l'un étant enfilé dans l'autre, par exemple la partie extrême 5a du tronçon de tube biopharmaceutique 5 étant placée autour et à l'extérieur du tronçon creux 7.

Dans une réalisation purement exemplative et non limitative, la paroi latérale périphérique constitutive du connecteur biopharmaceutique 4 présente des caractéristiques physico-chimiques identiques ou du moins fonctionnellement analogues à celles des parois 6 de la poche biopharmaceutique 2.

Au dispositif biopharmaceutique 1 peuvent être associés fonctionnellement et structurellement un ou plusieurs dispositifs d'arrêt d'écoulement du produit biopharmaceutique (non représenté) tel qu'une pince - amovible ou inamovible - (connue sous le nom de « clamp »), venant fermer la partie flexible du dispositif biopharmaceutique 1 où elle se trouve par aplatissement sur elle-même, plus spécialement le tronçon de tube 5 ou autre système de vidange en bas de poche.

Au dispositif biopharmaceutique 1 peuvent également être associés fonctionnellement et structurellement, le cas échéant, un ou plusieurs systèmes biopharmaceutiques de mélange, d'aération (pouvant être qualifiés de systèmes biopharmaceutiques fonctionnels)... (non représentés).

Le tube 5 dont il est question, qui peut également être désigné par conduit, conduite, tuyau, est un objet creux, allongé d'une certaine longueur finie, de forme générale cylindrique ou pseudo cylindrique, ouvert à une extrémité ou aux deux extrémités, apte à être empli du produit biopharmaceutique pour son mouvement (par exemple pour le transfert vers ou depuis une poche biopharmaceutique 2 ou entre deux poches 2) ou sa station (par exemple pour la constitution d'échantillon de produit pharmaceutique).

Le tube 5 est spécialement apte à l'emploi dans le dispositif biopharmaceutique 1, ce qui exclut un cathéter ou un dispositif analogue ou un tube de circulation extracorporelle du sang ou de passage de fluide lors d'une dialyse, d'une perfusion, ou d'une alimentation artificielle ou pour une destination et un usage analogues, relevant d'un autre domaine technique.

Le terme « tube » ici employé vise à la fois le cas où il est de grande longueur, telle que celle résultant de son processus de fabrication, et le cas où il est de longueur plus petite, telle que celle d'un tronçon provenant de la coupe réalisée dans une grande longueur et destiné à être incorporé au dispositif biopharmaceutique 1.

Le tube 5 comprend une paroi latérale périphérique 10 et limite un espace libre longitudinal 11 de transfert ou de station du produit biopharmaceutique. Selon une autre réalisation, non représentée, le tube 5 comprend plusieurs espaces libres longitudinaux parallèles, séparés par une ou plusieurs parois. C'est cet espace libre longitudinal 11 qui peut être fermé par aplatissement du tube biopharmaceutique 5 sur lui-même par une pince d'arrêt d'écoulement du produit biopharmaceutique.

Dans une réalisation purement exemplative et non limitative, le tube 5 est sécable au moyen d'un outil de coupe approprié. Il est en outre obturable, soit au moyen d'un dispositif d'arrêt d'écoulement tel qu'une pince comme indiqué précédemment soit par soudage ou pliage sur lui-même.

Dans des exemples non limitatifs, de tels dispositifs biopharmaceutiques 1 constituent un ensemble appelé poche de stockage et/ou remplissage et/ou manipulation et/ou transport et/ou mélange d'un produit biopharmaceutique ou un bioréacteur d'un produit biopharmaceutique.

De façon plus générale, le dispositif biopharmaceutique 1 objet de l'invention peut comporter plusieurs poches biopharmaceutiques 2, plusieurs accès 3, plusieurs tronçons de tubes biopharmaceutiques 5 (deux ou plus tronçons de tubes biopharmaceutiques pouvant le cas échéant être associés fonctionnellement et structurellement entre eux, bout à bout ou avec des dérivations), le cas échéant plusieurs connecteur biopharmaceutique 4, plusieurs dispositifs d'arrêt d'écoulement du produit biopharmaceutique, le cas échéant un ou plusieurs systèmes biopharmaceutiques de mélange, d'aération, et plus généralement systèmes biopharmaceutiques fonctionnels, agencés selon telle configuration adaptée à la destination et à l'application envisagées pour le dispositif biopharmaceutique 1.

Il est entendu que si le dispositif biopharmaceutique 1 pris dans son ensemble est destiné à l'élaboration, stockage, transport d'un produit biopharmaceutique et si tel élément constitutif 2, 4, 5 du dispositif biopharmaceutique 1 est destiné et apte à recevoir ce produit biopharmaceutique, l'invention inclus également le cas où tel élément constitutif 2, 4, 5 du dispositif biopharmaceutique 1 est destiné et apte à recevoir un ou plusieurs composants du produit biopharmaceutique ou le produit biopharmaceutique dans un ou plusieurs états distincts. C'est ainsi qu'il faut comprendre, notamment, que le tube biopharmaceutique 5 est destiné au transfert ou à la station du produit biopharmaceutique par l'espace libre longitudinal 11 ou que sa face intérieure limitant l'espace libre longitudinal est apte à être au contact du produit biopharmaceutique. L'invention vise toutes ces réalisations.

Le tube biopharmaceutique 5 est multicouche, sa paroi latérale périphérique 10 comportant une couche contact 12, interne, et une couche fonctionnelle 13, externe, comprenant au moins une couche élémentaire fonctionnelle 13i. Dans une réalisation, le tube biopharmaceutique 5 multicouche est constitué de la couche contact 12 et de la couche fonctionnelle 13.

La couche contact 12 constitue la face intérieure 14a du tube biopharmaceutique 5 multicouche qui limite l'espace libre longitudinal 11 et est apte à être au contact du produit biopharmaceutique. Elle est constituée d'une matière choisie apte à être au contact du produit biopharmaceutique sans dégradation de la couche contact et du produit biopharmaceutique et à être soudable sur elle-même, c'est-à-dire apte à assurer la fonction de contention et de séparation physique inhérente à un tube.

La matière constitutive de la couche contact 12 est choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE) le polyfluorure de vinylidène (PVDF). En aucun cas la matière constitutive de la couche contact 12 est du PVC.

La couche fonctionnelle 13 comprenant au moins une couche élémentaire fonctionnelle 13i est constituée d'une matière choisie, en fonction des besoins de la fonction et de la destination du dispositif biopharmaceutique 1, pour sa fonction de flexibilité, de robustesse, de manipulation, d'opacité ou au contraire de transparence, de barrière aux gaz non dégradable à elle seule ou par association à une couche protectrice, c'est-à-dire apte à assurer les fonctions permettant de satisfaire les autres propriétés physiques, chimiques, biologiques, opératoires adaptées à la destination et à l'application considérées du tube biopharmaceutique 5 multicouche et, plus généralement du dispositif biopharmaceutique 1 à usage unique.

La matière constitutive d'une couche élémentaire fonctionnelle 13i est autre que le PVC et choisie dans la famille comprenant le polyéthylène (PE) et notamment le polyéthylène à basse densité linéaire (LLDPE) ou le polyéthylène téréphtalate (PET), un polyamide (PA), le polyéthylène vinyle acétate (EVA), l'éthylène-alcool vinylique (EVOH), le styrène éthylène butadiène-styrène (SEBS), le polyéthylène téréphtalate glycol (PETG...), le polyfluorure de vinylidène (PVDF), dans le cadre de la destination et de l'application considérées.

Lorsque le tube biopharmaceutique 5 multicouche est constitué de la couche contact 12 et de la couche fonctionnelle 13, la couche fonctionnelle 13 forme la face extérieure 14b du tube 5.

Une solidarisation existe entre la face externe 15a de la couche contact 12 et la face interne 15b de la couche fonctionnelle 13, les deux couches 12 et 13 étant ainsi superposées. De même, une solidarisation existe entre la face externe et la face interne de deux couches fonctionnelles élémentaires 13i.

Le terme « superposé » appliqué à deux couches (ou éventuellement à deux agrégations de couches), comme le terme « superposition », doit être compris comme signifiant que ces couches (ou agrégations de couches) sont disposées l'une sur l'autre directement ou moyennant le cas échéant une couche d'interface de liaison, sans restriction s'agissant de celle des couches (ou agrégations de couches) située au dessus ou en dessous de l'autre, la position dans l'espace de l'ensemble formé par ces deux couches étant a *priori* quelconque.

Le terme « solidarisation » appliqué à deux couches (ou agrégations de couches) doit être compris comme signifiant que ces deux couches (ou agrégations de couches) forment un ensemble cohérent, soit directement soit, si la solidarisation mutuelle directe des deux couches (ou agrégations de couches) n'est pas possible, à raison des matériaux qui les constituent respectivement, moyennant une couche d'interface de liaison et d'association. L'homme du métier sait quels matériaux peuvent être solidarisés mutuellement directement et quels matériaux nécessitent une couche d'interface de liaison et d'association et le matériau constitutif de cette couche. Pour des raisons de simplification de la description, celle-ci ne mentionne pas l'éventuelle existence d'une telle couche d'interface de liaison et d'association, même si celle-ci est nécessaire et prévue, la présence d'une couche d'interface de liaison et d'association appropriée étant alors et en effet implicite et à la portée de l'homme du métier.

La superposition des couches contact 12 et fonctionnelle 13 constitutive de la paroi 10 du tube biopharmaceutique multicouche 5 permet que ce dernier remplisse les exigences attendues en ce qui concerne, d'une part, le tube 5 et, d'autre part, plus généralement, le dispositif biopharmaceutique 1 à usage unique.

La paroi 10 d'un tube biopharmaceutique 5 spécialement destiné et adapté à un dispositif biopharmaceutique 1 à usage unique doit être choisie et conçue, notamment quant à ses matières constitutives, ses formes, ses dimensions..., de sorte à présenter une certaine souplesse, être réalisée en matière plastique, et à être apte d'une part à avoir une propriété de contention du contenu du tube et de séparation physique entre celui-ci et l'extérieur du tube et, d'autre part, à avoir des propriétés physiques, chimiques, biologiques, opératoires adaptées à la destination et à l'application considérées. Des exemples non limitatifs de telles propriétés physiques, chimiques, biologiques, opératoires sont une propriété barrière à haut degré d'étanchéité aux gaz, l'aptitude d'un point de vue mécanique à contenir le produit biopharmaceutique se trouvant dans le tube 5, l'aptitude à éviter les interactions indésirables avec l'environnement ou le produit biopharmaceutique, par exemple lors de la fabrication, du transport ou du stockage, l'aptitude à être propre, notamment au regard des particules ou sous l'angle biologique, l'aptitude à être non dégradable et présenter une haute résistance chimique, l'aptitude, également, à permettre que l'élaboration ou la conservation du produit biopharmaceutique soit assurée avec sécurité, l'aptitude à être soudée, notamment sur un matériau identique ou analogue, l'aptitude à être pliée et dépliée, aisément et sans détérioration, l'aptitude à présenter des qualités appropriées au regard de l'adhérence ou de la souplesse. Selon les cas, avoir une certaine transparence ou au contraire une certaine opacité.

La superposition des couches contact 12 et fonctionnelle 13 constitutive de la paroi 10 du tube biopharmaceutique multicouche 5 permet que ce dernier remplisse les exigences attendues, d'une part, en ce qui concerne le tube 5 et, d'autre part, plus généralement, en ce qui concerne le dispositif biopharmaceutique 1 à usage unique.

La qualification de « fonctionnel » donnée à la couche 13 ou à la couche élémentaire 13i ne signifie pas, *a contrario,* que la couche contact 12 n'aurait aucune fonction. La qualification de « contact » donnée à la couche contact 12 et la qualification de « fonctionnel » donnée à la couche 13 signifient que la couche 12 assure essentiellement la fonction de contention du produit biopharmaceutique (ou d'un composant de celui-ci ou du produit biopharmaceutique dans un état particulier) et de séparation physique entre celui-ci et l'extérieur du tube, alors que la couche 13 assure dans son ensemble les fonctions permettant de satisfaire les autres propriétés physiques, chimiques, biologiques, opératoires adaptées à la destination et à l'application considérées, la - ou chaque - couche élémentaire 13i assurant l'une ou plusieurs des fonctions permettant de satisfaire les autres propriétés physiques, chimiques, biologiques, opératoires requises. La couche contact 12 assure donc bien, elle aussi une certaine fonction.

Alors que par nature la fonction de contention et de séparation physique est toujours assurée quel que soit le tube biopharmaceutique multicouche 5 considéré, les fonctions permettant de satisfaire les autres propriétés physiques, chimiques, biologiques, opératoires adaptées à la destination et à l'application considérées peuvent varier assez largement en fonction de ces destinations et de ces applications.

Il est donc possible d'envisager une couche contact 12 qui, en ce qui concerne ses matières constitutives, présente une certaine constance ou permanence dans la diversité des destinations et des applications envisagées pour le tube biopharmaceutique multicouche 5 et le dispositif biopharmaceutique 1 à usage unique. Pour la couche fonctionnelle 13, respectivement la - ou chaque - couche élémentaire 13i, il est possible d'envisager une variété de matières constitutives en fonction des autres propriétés physiques, chimiques, biologiques, opératoires requises.

Le choix des matières constitutives des couches contact 12 et fonctionnelle 13 est à la portée de l'homme du métier, selon les fonctions devant être assurées.

La solidarisation des deux couches 12 et 13 résulte du procédé de fabrication du tube biopharmaceutique multicouche 5 (co extrusion, enrobage, gainage...).

Les couche contact 12 et fonctionnelle 13 sont choisies, notamment quant à leurs formes, leurs dimensions..., de sorte à être aptes à assurer les fonctions de contention et de séparation physique et de conférer au tube biopharmaceutique 5 multicouche les propriétés physiques, chimiques, biologiques, opératoires adaptées à la destination et à l'application considérées. Ces choix sont à la portée de l'homme du métier.

Un procédé de fabrication d'un tel tube biopharmaceutique multicouche 5 est tel que l'on dispose de la matière choisie apte à constituer la couche contact 12, l'on dispose de la au moins une matière apte à constituer la au moins une couche élémentaire fonctionnelle 13i, l'on dispose de moyens opérationnels de machinabilité aptes à réaliser un tube multicouche, et l'on met en oeuvre ces moyens de machinabilité avec la matière choisie apte à constituer la couche contact 12 et la au moins une matière apte à constituer la au moins une couche élémentaire fonctionnelle 13i, de sorte que la face externe 15a de la couche contact 12 et la face interne 15b de la couche fonctionnelle 13 soient solidarisées l'une avec l'autre.

Dans une première réalisation exemplative, le procédé de fabrication du tube biopharmaceutique multicouche 5 est à fonctionnement en continu, en une ou plusieurs étapes, le caractère multicouche étant réalisé notamment par co extrusion ou enrobage.

Dans une seconde réalisation exemplative, le procédé de fabrication est à fonctionnement en discontinu, portant sur un tronçon de tube, le caractère multicouche étant réalisé notamment par gainage d'un tronçon de tube constitué par la couche contact 12 dans un tronçon d'un tube ou de segments de tubes ouvert(s) longitudinalement constitué par la couche fonctionnelle 13 ou une couche élémentaire fonctionnelle 13i.

La poche biopharmaceutique 2 et le cas échéant un connecteur biopharmaceutique 4 comporte, de façon analogue au (ou aux) tube(s) biopharmaceutique(s) multicouche(s) 5, une couche contact 16 constituée d'une matière choisie apte à être au contact du produit biopharmaceutique sans dégradation de la couche contact et du produit biopharmaceutique et à être soudable sur elle-même.

La matière de la couche contact 16 de la poche biopharmaceutique 2 et le cas échéant du connecteur biopharmaceutique 4 est choisie identique ou analogue à la matière de la couche contact 12 du tube biopharmaceutique multicouche 5.

La couche contact 16 constitue la face intérieure de la poche biopharmaceutique 2 et le cas échéant du connecteur biopharmaceutique 4 et elle est apte à assurer la fonction de contention et de séparation physique inhérente à cette poche 2 et ce connecteur 4.

La matière de la couche contact 16 est choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE), le polyfluorure de vinylidène (PVDF) et les matériaux équivalents, dans le cadre de la destination et de l'application considérées. En aucun cas la matière formant la couche contact 16 est du PVC.

Dans une réalisation exemplative et non limitative, la poche biopharmaceutique 2 et le cas échéant un connecteur biopharmaceutique 4 est multicouche et a une structure multicouche identique ou analogue à la structure multicouche du (ou des) tube(s) biopharmaceutique(s) multicouche(s) 5. Dans ce cas, la poche biopharmaceutique 2 et le cas échéant un connecteur biopharmaceutique 4 comprend, outre la couche contact 16, une couche fonctionnelle 17 comprenant au moins une couche élémentaire fonctionnelle 17i.

La couche fonctionnelle 17, respectivement la couche élémentaire fonctionnelle 17i est constituée d'une matière choisie, en fonction des besoins de la fonction et de la destination du dispositif biopharmaceutique 1, pour sa fonction de flexibilité, de robustesse, de manipulation, d'opacité ou au contraire de transparence, de barrière aux gaz non dégradable à elle seule ou par association à une couche protectrice, c'est-à-dire apte à assurer les fonctions permettant de satisfaire les autres propriétés physiques, chimiques, biologiques, opératoires adaptées à la destination et à l'application considérées du dispositif biopharmaceutique 1 à usage unique. Cette matière est autre que le PVC.

Dans le dispositif biopharmaceutique 1 monté, la couche contact 12 d'un tube biopharmaceutique multicouche 5 est soudée à la couche contact 16 de la poche biopharmaceutique 2, le cas échéant d'un autre tronçon de tube biopharmaceutique et/ou du connecteur biopharmaceutique 4, selon les configurations et les destinations et applications, avec une continuité physique substantielle entre les couches contact 12 et 16 respectives.

Dans une réalisation exemplative et non limitative, dans le cas où la poche biopharmaceutique 2, le cas échéant un connecteur biopharmaceutique 4, est multicouche, comme décrit, la couche fonctionnelle 13 d'un tube biopharmaceutique multicouche 5 est par ailleurs soudée à la couche fonctionnelle 17 de la poche biopharmaceutique 2, le cas échéant d'un autre tronçon de tube biopharmaceutique et/ou du connecteur biopharmaceutique 4, notamment avec une continuité physique substantielle entre les couches fonctionnelles 13 et 17 respectives.

Pour fabriquer un dispositif biopharmaceutique 1 à usage unique tel que décrit, on dispose d'au moins une poche biopharmaceutique 2, d'au moins un tronçon de tube biopharmaceutique 5 et, le cas échéant, d'au moins un connecteur biopharmaceutique 5.

Puis, on positionne ces éléments dans la configuration du dispositif biopharmaceutique 1 à fabriquer.

Puis on soude ces éléments entre eux de façon communicante, continue, rigide et étanche, de sorte que la couche contact 12 d'un tube biopharmaceutique 5 soit soudée à la couche contact 16 de la poche biopharmaceutique 2, le cas échéant d'un autre tronçon de tube biopharmaceutique et/ou du connecteur biopharmaceutique 4 avec une continuité physique substantielle entre les couches contact respectives 12 et 16, comme indiqué précédemment.

L'invention a pour objet non seulement un dispositif biopharmaceutique 1 à usage unique destiné à l'élaboration, au stockage et au transport d'un produit biopharmaceutique, comme décrit mais également un tube biopharmaceutique multicouche 5 spécialement adapté et destiné à un tel dispositif biopharmaceutique 1 à usage unique et comportant une couche contact 12 et une couche fonctionnelle 13 comprenant au moins une couche élémentaire fonctionnelle 13i, solidarisées entre elles.

Selon les réalisations, un tel tube biopharmaceutique multicouche 5 peut être à section transversale intérieure et/ou extérieure circulaire ou oblongue. Il peut comporter un espace libre longitudinal 11 unique ou plusieurs espaces libres longitudinaux juxtaposés avec séparation étanche. Il peut être homogène d'une extrémité à l'autre du tronçon qu'il forme ou comporter à au moins une partie extrême 5a du tronçon qu'il forme une partie saillante découverte constituée de la couche de contact 12, notamment en forme de collerette transversale dirigée vers l'extérieur, un tel tronçon de tube biopharmaceutique multicouche 5 faisant alors également fonction de connecteur pour le dispositif biopharmaceutique 1 à usage unique. Un tel tube-connecteur peut être associé à une poche biopharmaceutique 1 ou, le cas échéant à un autre tube biopharmaceutique 5.

Sur la figure 4A est illustrée une réalisation dans laquelle le tube biopharmaceutique multicouche 5 comprend une unique couche élémentaire fonctionnelle 13, 13a, la couche fonctionnelle 13 étant alors monocouche. Par exemple, le tube biopharmaceutique multicouche 5 comporte, associé à une couche contact 12 de LLDPE, une unique couche élémentaire fonctionnelle 13, 13a de PE.

Dans d'autres réalisations, le tube biopharmaceutique multicouche 5 comprend une pluralité de couches élémentaires fonctionnelles 13i, 13j..., chacune solidarisée à la ou aux couches adjacentes, la couche fonctionnelle 13 étant alors multicouche.

Le terme « adjacent » appliqué à deux couches (ou agrégations de couches) doit être compris comme signifiant que ces deux couches (ou agrégations de couches) sont contigües, directement ou moyennant le cas échéant une interface de liaison.

Sur la figure 4B est illustrée une réalisation dans laquelle la paroi 10 du tube biopharmaceutique multicouche 5 comporte, associées à une couche contact 12 de LLDPE, une couche élémentaire fonctionnelle 13a d'EVOH, une couche élémentaire fonctionnelle 13b de PA et une couche élémentaire fonctionnelle 13c de PET qui forme la face extérieure du tube biopharmaceutique multicouche. Ici, la couche élémentaire fonctionnelle 13a d'EVOH est solidarisée à la couche contact 12 de LLDPE, tandis que la couche élémentaire fonctionnelle 13c de PET forme la face extérieure 14b du tube biopharmaceutique multicouche 5.

Sur la figure 4C est illustrée une réalisation dans laquelle la paroi 10 du tube biopharmaceutique multicouche 5 comporte, associées à une couche contact 12 de LLDPE, une couche élémentaire fonctionnelle 13a d'EVOH, une couche élémentaire fonctionnelle 13b de PA, deux couches élémentaires fonctionnelles 13c de LLDPE et une couche élémentaire fonctionnelle 13d en matériau biodégradable. Ici, la couche élémentaire fonctionnelle 13a d'EVOH est solidarisée à la couche contact 12 de LLDPE, tandis que la couche élémentaire fonctionnelle 13d en matériau biodégradable forme la face extérieure 14b du tube biopharmaceutique multicouche 5.

Sur la figure 4D est illustrée une réalisation dans laquelle la paroi 10 du tube biopharmaceutique multicouche 5 comporte, associées à une couche contact 12 de LLDPE, une couche élémentaire fonctionnelle 13a en matériau biodégradable, deux couches élémentaires fonctionnelles 13b de LLDPE, une couche élémentaire fonctionnelle 13c d'EVOH et une couche élémentaire fonctionnelle 13d de PA. Ici, la couche élémentaire fonctionnelle 13a en matériau biodégradable est solidarisée à la couche contact 12 de LLDPE, tandis que la couche élémentaire fonctionnelle 13d de PA forme la face extérieure 14b du tube biopharmaceutique multicouche 5.

Sur la figure 4E est illustrée une réalisation dans laquelle la paroi 10 du tube biopharmaceutique multicouche 5 comporte, associées à une couche contact 12 de PE, une couche élémentaire fonctionnelle 13a d'EVA, une couche élémentaire fonctionnelle 13b d'EVOH, une couche élémentaire fonctionnelle 13c d'EVA et une couche élémentaire fonctionnelle 13d de PE. Ici, la couche élémentaire fonctionnelle 13a d'EVA est solidarisée à la couche contact 12 de PE, tandis que la couche élémentaire fonctionnelle 13d de PE forme la face extérieure 14b du tube biopharmaceutique multicouche 5.

Sur la figure 4F est illustrée une réalisation dans laquelle la paroi 10 du tube biopharmaceutique multicouche 5 comporte, associées à une couche contact 12 de LLDPE, une couche élémentaire fonctionnelle 13a d'EVOH, une couche élémentaire fonctionnelle 13b de LLDPE et une couche élémentaire fonctionnelle 13c de PE. Ici, la couche élémentaire fonctionnelle 13a d'EVOH est solidarisée à la couche contact 12 de PE, tandis que la couche élémentaire fonctionnelle 13c de PE forme la face extérieure 14b du tube biopharmaceutique multicouche 5.

## Revendications

1. Dispositif biopharmaceutique à usage unique (1) d'élaboration, stockage, transport de produit biopharmaceutique, comprenant au moins un tronçon de tube biopharmaceutique (5) multicouche, ayant une paroi latérale périphérique (10) limitant un espace libre longitudinal (11) et comportant une couche contact et une couche fonctionnelle avec une solidarisation entre la face externe (15a) de la couche contact (12) et la face interne (15b) de la couche fonctionnelle (13), au moins une poche biopharmaceutique (2), et, le cas échéant, au moins un connecteur biopharmaceutique (4), chacun à usage unique, et comportant une couche contact (16) constituée d'une matière choisie apte à être au contact du produit biopharmaceutique sans dégradation de la couche contact (16) et du produit biopharmaceutique et à être soudable sur elle-même, soudés entre eux de façon communicante, continue, rigide et étanche, présentant une certaine souplesse et réalisés en matière plastique,
**caractérisé en ce que** :
- la couche contact (12) du tube biopharmaceutique (5) est constituée d'une matière choisie apte à être au contact du produit biopharmaceutique sans dégradation de la couche contact (12) et du produit biopharmaceutique et à être soudable sur elle-même, autre que le PVC et choisie dans la famille comprenant le polyéthylène (PE) et notamment le polyéthylène à basse densité linéaire (LLDPE), le polyéthylène vinyle acétate (EVA), le polypropylène (PP), l'éthylène-tétrafluoroéthylène (ETFE) le polyfluorure de vinylidène (PVDF), dont la face intérieure (14a) limite l'espace libre longitudinal (11) et est apte à être au contact du produit biopharmaceutique, c'est-à-dire est apte essentiellement à assurer la fonction de contention et de séparation physique du au moins un tube biopharmaceutique (5) et formant la face intérieure (14a) de celui-ci,
- la couche fonctionnelle (13) du tube biopharmaceutique (5) comprend au moins une couche élémentaire fonctionnelle (13i) constituée d'une matière choisie pour sa fonction de flexibilité, de robustesse, de manipulation, d'opacité ou au contraire de transparence, de barrière aux gaz, non dégradable à elle seule ou par association à une couche protectrice, autre que le PVC et choisie dans la famille comprenant le polyéthylène (PE) et notamment le polyéthylène à basse densité linéaire (LLDPE), le polyéthylène téréphtalate (PET), un polyamide (PA), le polyéthylène vinyle acétate (EVA), l'éthylène-alcool vinylique (EVOH), le styrène éthylène butadiène-styrène (SEBS), le polyéthylène téréphtalate glycol (PETG...), le polyfluorure de vinylidène (PVDF), c'est-à-dire apte à assurer les fonctions permettant de satisfaire les autres propriétés physiques, chimiques, biologiques, opératoires adaptées à la destination et à l'application considérées du dispositif biopharmaceutique (1) à usage unique,
- la couche contact (16) de la poche (2), d'un autre tronçon de tube (5) et/ou d'un connecteur (4), est constituée d'une matière choisie identique ou analogue à la matière de la couche contact (12) du au moins un tube biopharmaceutique (5),
- la couche contact (12) du tube biopharmaceutique (5) est soudée à la couche contact (16) de la poche (2), d'un autre tronçon de tube (5) et/ou d'un connecteur (4), avec une continuité physique substantielle entre les couches contact respectives (12, 16).

2. Dispositif biopharmaceutique à usage unique (1) d'élaboration, stockage, transport de produit biopharmaceutique selon la revendication 1, **caractérisé en ce qu'**une poche biopharmaceutique (2), le cas échéant un autre tronçon de tube biopharmaceutique (5) et/ou un connecteur biopharmaceutique (4), soudé au au moins un tube biopharmaceutique (5), a une structure multicouche identique ou analogue à la structure multicouche du au moins un tube biopharmaceutique (5) multicouche, à savoir comprenant une couche contact (16) et une couche fonctionnelle (17) comprenant au moins une couche élémentaire fonctionnelle (17i).

3. Dispositif biopharmaceutique à usage unique (1) d'élaboration, stockage, transport de produit biopharmaceutique selon la revendication 2, **caractérisé en ce que** la couche fonctionnelle (13) du au moins un tube biopharmaceutique (5) est soudée à la couche fonctionnelle (17) de la poche biopharmaceutique (2), le cas échéant de l'autre tronçon de tube biopharmaceutique (5) et/ou du connecteur biopharmaceutique (4).

4. Dispositif biopharmaceutique à usage unique (1) d'élaboration, stockage, transport de produit biopharmaceutique selon l'une quelconque des revendications 1 à 3, présentant au moins l'une des caractéristiques suivantes :
- le au moins un tube biopharmaceutique (5) est soudé à un autre élément constitutif du dispositif biopharmaceutique (1) soit directement formant ainsi intrinsèquement connecteur biopharmaceutique (4) soit indirectement par l'intermédiaire d'un connecteur biopharmaceutique (4) ;
- il est associé structurellement et fonctionnellement au dispositif biopharmaceutique à usage unique, de façon amovible ou inamovible, au moins un dispositif d'arrêt d'écoulement du produit biopharmaceutique dans l'espace libre longitudinal (11) du au moins un tube biopharmaceutique (5) ayant la forme d'une pince apte à fermer la partie du au moins un tube biopharmaceutique (5) où elle se trouve par aplatissement du moins un tube biopharmaceutique (5) sur lui-même ;
- il constitue une poche de stockage et/ou remplissage et/ou manipulation et/ou transport et/ou mélange d'un produit biopharmaceutique ou un bioréacteur d'un produit biopharmaceutique.

5. Dispositif biopharmaceutique à usage unique (1) d'élaboration, stockage, transport de produit biopharmaceutique selon l'une quelconque des revendications précédentes, **caractérisé en ce que** ledit tube biopharmaceutique (5) est constitué de la couche contact (12) et de la couche fonctionnelle (13), la couche fonctionnelle (13) formant la face extérieure (14b) du tube biopharmaceutique (5) multicouche.

6. Dispositif biopharmaceutique à usage unique (1) d'élaboration, stockage, transport de produit biopharmaceutique selon l'une quelconque des revendications précédentes, **caractérisé en ce que** le tube biopharmaceutique (5) comprend une unique couche élémentaire fonctionnelle (13i).

7. Dispositif biopharmaceutique à usage unique (1) d'élaboration, stockage, transport de produit biopharmaceutique selon l'une quelconque des revendications 1 à 6, **caractérisé en ce que** le tube biopharmaceutique (5) comprend une pluralité de couches élémentaires fonctionnelles (13i), chacune solidarisée à la ou aux couches adjacentes.

8. Dispositif biopharmaceutique à usage unique (1) d'élaboration, stockage, transport de produit biopharmaceutique selon l'une quelconque des revendications précédentes, **caractérisé en ce que** la solidarisation entre la face externe (15a) de la couche contact (12) et la face interne (15b) de la couche fonctionnelle (13) est assurée de fabrication, par co extrusion ou enrobage ou gainage.

9. Dispositif biopharmaceutique à usage unique (1) d'élaboration, stockage, transport de produit biopharmaceutique selon la revendication 6, **caractérisé en ce que** le tube biopharmaceutique (5) comporte, associée à une couche contact (12) de LLDPE, une couche élémentaire fonctionnelle (13a) de PE.

10. Dispositif biopharmaceutique à usage unique (1) d'élaboration, stockage, transport de produit biopharmaceutique selon la revendication 7, **caractérisé en ce que** le tube biopharmaceutique (5) comporte l'une des caractéristiques suivantes :
- associées à une couche contact (12) de LLDPE, une couche élémentaire fonctionnelle (13a) d'EVOH, une couche élémentaire fonctionnelle (13b) de PA et une couche élémentaire fonctionnelle (13c) de PET qui forme la face extérieure (14b) du tube biopharmaceutique (5) multicouche ;
- associées à une couche contact (12) de LLDPE, une couche élémentaire fonctionnelle (13a) d'EVOH, une couche élémentaire fonctionnelle (13b) de PA, deux couches élémentaires fonctionnelles de LLDPE (13c) et une couche élémentaire fonctionnelle (13d) en matériau biodégradable qui forme la face extérieure (14b) du tube biopharmaceutique (5) multicouche ;
- associées à une couche contact (12) de LLDPE, une couche élémentaire fonctionnelle (13a) en matériau biodégradable, deux couches élémentaires fonctionnelles de LLDPE (13b), une couche élémentaire fonctionnelle (13c) d'EVOH et une couche élémentaire fonctionnelle (13d) de PA qui forme la face extérieure (14b) du tube biopharmaceutique (5) multicouche ;
- associées à une couche contact (12) de PE, une couche élémentaire fonctionnelle (13a) d'EVA, une couche élémentaire fonctionnelle (13b) d'EVOH, une couche élémentaire fonctionnelle (13c) d'EVA et une couche élémentaire fonctionnelle de PE (13d) qui forme la face extérieure (14b) du tube biopharmaceutique (5) multicouche ;
- associées à une couche contact (12) de LLDPE, une couche élémentaire fonctionnelle (13a) d'EVOH, une couche élémentaire fonctionnelle (13b) de LLDPE et une couche élémentaire fonctionnelle (13c) de PE qui forme la face extérieure du tube biopharmaceutique (5) multicouche.

11. Dispositif biopharmaceutique à usage unique (1) d'élaboration, stockage, transport de produit biopharmaceutique selon l'une quelconque des revendications précédentes, **caractérisé en ce que** le tube biopharmaceutique (5) comporte au moins l'une des caractéristiques suivantes :
- il est à section transversale circulaire ou oblongue ;
- il comporte un espace libre longitudinal unique ou plusieurs espaces libres longitudinaux juxtaposés avec séparation étanche.

12. Dispositif biopharmaceutique à usage unique (1) d'élaboration, stockage, transport de produit biopharmaceutique selon l'une quelconque des revendications précédentes, **caractérisé par le fait que** le tube biopharmaceutique (5) est homogène d'une extrémité à l'autre du tronçon qu'il forme.

13. Dispositif biopharmaceutique à usage unique (1) d'élaboration, stockage, transport de produit biopharmaceutique selon l'une quelconque des revendications précédentes, **caractérisé par le fait que** le tube biopharmaceutique (5) comporte à au moins une extrémité du tronçon qu'il forme une partie saillante découverte constituée de la couche de contact, un tel tronçon de tube biopharmaceutique (5) multicouche faisant également fonction de connecteur pour le dispositif biopharmaceutique à usage unique (1) d'élaboration, stockage, transport de produit biopharmaceutique.

14. Procédé de fabrication d'un dispositif biopharmaceutique à usage unique (1) d'élaboration, stockage, transport de produit biopharmaceutique selon l'une quelconque des revendications 1 à 13, dans lequel on dispose d'au moins une poche biopharmaceutique (2), d'au moins un tronçon de tube biopharmaceutique (5) et, le cas échéant, d'au moins un connecteur biopharmaceutique (4), et on positionne ces éléments dans la configuration du dispositif biopharmaceutique à fabriquer et on les soude entre eux de façon communicante, continue, rigide et étanche, de sorte que la couche contact (12) du au moins un tube biopharmaceutique (5) soit soudée à la couche contact (16) de la poche biopharmaceutique (2), d'un autre tronçon de tube biopharmaceutique (5) ou le cas échéant du connecteur biopharmaceutique (4) avec une continuité physique substantielle entre les couches contact respectives (12, 16).

## Patentansprüche

1. Biopharmazeutische Vorrichtung zur einmaligen Verwendung (1) für Produktion, Lagerung, Transport eines biopharmazeutischen Produkts, umfassend mindestens einen Abschnitt eines mehrschichtigen biopharmazeutischen Schlauchs (5) mit einer peripheren Seitenwand (10), die einen länglichen Freiraum (11) begrenzt und eine Kontaktschicht und eine Funktionsschicht mit einer festen Verbindung zwischen der äußeren Fläche (15a) der Kontaktschicht (12) und der inneren Fläche (15b) der Funktionsschicht (13), mindestens einen biopharmazeutischen Beutel (2) und gegebenenfalls mindestens einen biopharmazeutischen Verbinder (4), jeweils zur einmaligen Verwendung, umfasst und eine Kontaktschicht (16) aufweist, die aus einem ausgewählten Material besteht, das imstande ist, mit dem biopharmazeutischen Produkt ohne Beschädigung der Kontaktschicht (16) und des biopharmazeutischen Produkts im Kontakt zu sein und mit sich selbst verschweißbar zu sein, die kommunizierend, kontinuierlich, fest und dicht miteinander verschweißt sind, eine bestimmte Elastizität aufweisen und aus Kunststoffmaterial hergestellt sind,
**dadurch gekennzeichnet, dass**:
- die Kontaktschicht (12) des biopharmazeutischen Schlauchs (5) aus einem Material besteht, das imstande ist, mit dem biopharmazeutischen Produkt ohne Beschädigung der Kontaktschicht (12) und des biopharmazeutischen Produkts im Kontakt zu sein und mit sich selbst verschweißbar zu sein, das kein PVC ist und aus der Familie ausgewählt ist, die Polyethylen (PE) und insbesondere lineares Polyethylen niederer Dichte (LLDPE), Polyethylenvinylacetat (EVA), Polypropylen (PP), Ethylen-Tetrafluorethylen (ETFE), Polyvinylidenfluorid (PVDF) umfasst, dessen untere Fläche (14a) den länglichen Freiraum (11) begrenzt und imstande ist, mit dem biopharmazeutischen Produkt im Kontakt zu sein, das heißt, im Wesentlichen imstande ist, die Funktion des Enthaltens und des physischen Trennens von dem mindestens einen biopharmazeutischen Schlauch (5) zu sichern und die untere Fläche (14a) desselben bildet,
- die Funktionsschicht (13) des biopharmazeutischen Schlauchs (5) mindestens eine elementare Funktionsschicht (13i) umfasst, die aus einem Material besteht, das wegen seiner Flexibilitäts-, Robustheits-, Handhabbarkeitsfunktion, Lichtdichte oder, im Gegenteil, Lichtdurchlässigkeit, Gasbarrierefunktion ausgewählt ist, das allein oder durch Kombination mit einer Schutzschicht nicht abbaubar ist, das kein PVC ist und aus der Familie ausgewählt ist, die Polyethylen (PE) und insbesondere lineares Polyethylen niederer Dichte (LLDPE), Polyethylenterephthalat (PET), ein Polyamid (PA), Polyethylenvinylacetat (EVA), Ethylenvinylalkohol (EVOH), Styrol-Ethylen-Butadien-Styrol (SEBS), Polyethylenterephthalatglycol (PETG...), Polyvinylidenfluorid (PVDF) umfasst, das heißt, imstande ist, die Funktionen zu sichern, die erlauben, die anderen physikalischen, chemischen, biologischen, operativen Eigenschaften zu erfüllen, die an den berücksichtigten Bestimmungszweck und Anwendung der biopharmazeutischen Vorrichtung (1) zur einmaligen Verwendung angepasst sind,
- die Kontaktschicht (16) des Beutels (2), eines anderen Abschnitts des Schlauchs (5) und/oder eines Verbinders (4) aus einem Material besteht, das identisch oder analog zum Material der Kontaktschicht (12) des mindestens einen biopharmazeutischen Schlauchs (5) ausgewählt ist,
- die Kontaktschicht (12) des biopharmazeutischen Schlauchs (5) mit der Kontaktschicht (16) des Beutels (2), eines anderen Abschnitts des Schlauchs (5) und/oder eines Verbinders (4) mit einer substantiellen physischen Kontinuität zwischen den jeweiligen Kontaktschichten (12, 16) verschweißt ist.

2. Biopharmazeutische Vorrichtung zur einmaligen Verwendung (1) für Produktion, Lagerung, Transport eines biopharmazeutischen Produkts nach Anspruch 1, **dadurch gekennzeichnet, dass** ein biopharmazeutischer Beutel (2), gegebenenfalls ein anderer Abschnitt des biopharmazeutischen Schlauchs (5) und/oder eines biopharmazeutischen Verbinders (4), der mit mindestens einem biopharmazeutischen Schlauch (5) verschweißt ist, eine Mehrschichtstruktur hat, die mit der Mehrschichtstruktur des mindestens einen mehrschichtigen biopharmazeutischen Schlauchs (5) identisch oder dazu analog ist, nämlich eine Kontaktschicht (16) und eine Funktionsschicht (17) umfasst, die mindestens eine elementare Funktionsschicht (17i) umfasst.

3. Biopharmazeutische Vorrichtung zur einmaligen Verwendung (1) für Produktion, Lagerung, Transport eines biopharmazeutischen Produkts nach Anspruch 2, **dadurch gekennzeichnet, dass** die Funktionsschicht (13) des mindestens einen biopharmazeutischen Schlauchs (5) mit der Funktionsschicht (17) des biopharmazeutischen Beutels (2), gegebenenfalls des anderen Abschnitts des biopharmazeutischen Schlauchs (5) und/oder des biopharmazeutischen Verbinders (4) verschweißt ist.

4. Biopharmazeutische Vorrichtung zur einmaligen Verwendung (1) für Produktion, Lagerung, Transport eines biopharmazeutischen Produkts nach einem der Ansprüche 1 bis 3, die mindestens eins der folgenden Merkmale aufweist:
- der mindestens eine biopharmazeutische Schlauch (5) ist mit einem anderen konstitutiven Element der biopharmazeutischen Vorrichtung (1) entweder direkt, wobei somit der biopharmazeutische Verbinder (4) intrinsisch gebildet wird, oder indirekt über einen biopharmazeutischen Verbinder (4) verschweißt;
- der biopharmazeutischen Vorrichtung zur einmaligen Verwendung ist strukturell und funktionell lösbar oder unlösbar mindestens eine Vorrichtung zugeordnet, die das Fließen des biopharmazeutischen Produkts im länglichen Freiraum (11) des mindestens einen biopharmazeutischen Schlauchs (5) stoppt, die die Form einer Klemme hat, die imstande ist, den Teil des mindestens einen biopharmazeutischen Schlauchs (5), wo sie sich befindet, durch Abflachen des mindestens einen biopharmazeutischen Schlauchs (5) auf sich selbst zu verschließen;
- sie bildet einen Beutel für die Lagerung und/oder das Befüllen und/oder die Handhabung und/oder den Transport und/oder das Mischen eines biopharmazeutischen Produkts oder einen Bioreaktor eines biopharmazeutischen Produkts.

5. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** der biopharmazeutische Schlauch (5) aus der Kontaktschicht (12) und der Funktionsschicht (13) besteht, wobei die Funktionsschicht (13) die äußere Fläche (14b) des mehrschichtigen biopharmazeutischen Schlauchs (5) bildet.

6. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** der biopharmazeutische Schlauch (5) eine einzige elementare Funktionsschicht (13i) umfasst.

7. Biopharmazeutische Vorrichtung zur einmaligen Verwendung (1) für Produktion, Lagerung, Transport eines biopharmazeutischen Produkts nach einem der Ansprüche 1 bis 6, **dadurch gekennzeichnet, dass** der biopharmazeutische Schlauch (5) eine Vielzahl elementarer Funktionsschichten (13i) umfasst, die jeweils mit der oder den benachbarten Schichten fest verbunden sind.

8. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** die feste Verbindung zwischen der äußeren Fläche (15a) der Kontaktschicht (12) und der inneren Fläche (15b) der Funktionsschicht (13) bei der Herstellung durch Co-Extrusion oder Umhüllung oder Ummantelung sichergestellt wird.

9. Biopharmazeutische Vorrichtung zur einmaligen Verwendung (1) für Produktion, Lagerung, Transport eines biopharmazeutischen Produkts nach Anspruch 6, **dadurch gekennzeichnet, dass** der biopharmazeutische Schlauch (5), der einer Kontaktschicht (12) aus LLDPE zugeordnet ist, eine elementare Funktionsschicht (13a) aus PE aufweist.

10. Biopharmazeutische Vorrichtung zur einmaligen Verwendung (1) für Produktion, Lagerung, Transport eines biopharmazeutischen Produkts nach Anspruch 7, **dadurch gekennzeichnet, dass** der biopharmazeutische Schlauch (5) eins der folgenden Merkmale aufweist:
- einer Kontaktschicht (12) aus LLDPE zugeordnet, eine elementare Funktionsschicht (13a) aus EVOH, eine elementare Funktionsschicht (13b) aus PA und eine elementare Funktionsschicht (13c) aus PET, die die äußere Fläche (14b) des mehrschichtigen biopharmazeutischen Schlauchs (5) bildet;
- einer Kontaktschicht (12) aus LLDPE zugeordnet, eine elementare Funktionsschicht (13a) aus EVOH, eine elementare Funktionsschicht (13b) aus PA, zwei elementare Funktionsschichten (13c) aus LLDPE und eine elementare Funktionsschicht (13d) aus biologisch abbaubarem Material, die die äußere Fläche (14b) des mehrschichtigen biopharmazeutischen Schlauchs (5) bildet,
- einer Kontaktschicht (12) aus LLDPE zugeordnet, eine elementare Funktionsschicht (13a) aus biologisch abbaubarem Material, zwei elementare Funktionsschichten (13b) aus LLDPE, eine elementare Funktionsschicht (13c) aus EVOH und eine elementare Funktionsschicht (13d) aus PA, die die äußere Fläche (14b) des mehrschichtigen biopharmazeutischen Schlauchs (5) bildet;
- einer Kontaktschicht (12) aus PE zugeordnet, eine elementare Funktionsschicht (13a) aus EVA, eine elementare Funktionsschicht (13b) aus EVOH, eine elementare Funktionsschicht (13c) aus EVA und eine elementare Funktionsschicht (13d) aus PE, die die äußere Fläche (14b) des mehrschichtigen biopharmazeutischen Schlauchs (5) bildet;
- einer Kontaktschicht (12) aus LLDPE zugeordnet, eine elementare Funktionsschicht (13a) aus EVOH, eine elementare Funktionsschicht (13b) aus LLDPE und eine elementare Funktionsschicht (13c) aus PE, die die äußere Fläche des mehrschichtigen biopharmazeutischen Schlauchs (5) bildet.

11. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** der biopharmazeutische Schlauch (5) mindestens eins der folgenden Merkmale aufweist:
- sein Querschnitt ist rund oder länglich;
- er weist einen einzigen länglichen Freiraum oder mehrere nebeneinanderliegende längliche Freiräume mit dichter Trennung auf.

12. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** der biopharmazeutische Schlauch (5) von einem Ende zum anderen des Abschnitts, den er bildet, homogen ist.

13. Biopharmazeutische Vorrichtung zur einmaligen Verwendung (1) für Produktion, Lagerung, Transport eines biopharmazeutischen Produkts nach einem der vorangehenden Ansprüche, **dadurch gekennzeichnet, dass** der biopharmazeutische Schlauch (5) an mindestens einem Ende des Abschnitts, den er bildet, einen abgedeckten hervorstehenden Teil aufweist, der aus der Kontaktschicht gebildet ist, wobei ein derartiger Abschnitt des mehrschichtigen biopharmazeutischen Schlauchs (5) ebenfalls als Verbinder für die biopharmazeutische Vorrichtung zur einmaligen Verwendung (1) für Produktion, Lagerung, Transport eines biopharmazeutischen Produkts dient.

14. Verfahren zur Herstellung einer biopharmazeutischen Vorrichtung zur einmaligen Verwendung (1) für Produktion, Lagerung, Transport eines biopharmazeutischen Produkts nach einem der Ansprüche 1 bis 13, wobei man mindestens über einen biopharmazeutischen Beutel (2), mindestens einen Abschnitt eines biopharmazeutischen Schlauchs (5) und gegebenenfalls mindestens einen biopharmazeutischen Verbinder (4) verfügt, und diese Elemente in der Konfiguration der herzustellenden biopharmazeutischen Vorrichtung positioniert werden und sie kommunizierend, kontinuierlich, fest und dicht derart miteinander verschweißt werden, das die Kontaktschicht (12) des mindestens einen biopharmazeutischen Schlauchs (5) mit der Kontaktschicht (16) des biopharmazeutischen Beutels (2), eines anderen Abschnitts des biopharmazeutischen Schlauchs (5) oder gegebenenfalls des biopharmazeutischen Verbinders (4) mit einer substantiellen physischen Kontinuität zwischen den jeweiligen Kontaktschichten (12, 16) verschweißt ist.

## Claims

1. A single-use biopharmaceutical device (1) for producing, storing, transporting biopharmaceutical product, comprising at least one multilayer segment of biopharmaceutical tube (5), having a peripheral side wall (10) limiting a longitudinal empty space (11) and including a contact layer and a functional layer with the outer face (15a) of the contact layer (12) and the inner face (15b) of the functional layer (13) being secured together, at least one biopharmaceutical bag (2), and, optionally, at least one biopharmaceutical connector (4), each being single use, and having a contact layer (16) made of a material chosen to be suitable for being in contact with the biopharmaceutical product without resulting in degradation of the contact layer (16) and of the biopharmaceutical product and for being weldable to itself, welded together in communicating, continuous, rigid and leaktight manner, having a certain amount of flexibility and made of plastic,
**characterized in that**:
- the contact layer (12) of the biopharmaceutical tube (5) is made of a material chosen to be suitable for being in contact with the biopharmaceutical product without degradation of the contact layer (12) and of the biopharmaceutical product and of being weldable on itself, other than PVC and chosen from the family comprising polyethylene (PE) and in particular linear low-density polyethylene (LLDPE), polyethylene vinyl acetate (EVA), polypropylene (PP), ethylene-tetrafluoroethylene (ETFE) polyvinylidene fluoride (PVDF), the inner face (14a) of which limits the longitudinal empty space (11) and is suitable for being in contact with the biopharmaceutical product, i.e. is suitable essentially for performing the containing and physical separation function of the at least one biopharmaceutical tube (5) and forming the inner face (14a) thereof,
- the functional layer (13) of the biopharmaceutical tube (5) comprises at least one functional elementary layer (13i) made of a material chosen for its function of flexibility, robustness, handling, opacity or, conversely, transparence, barrier to gases, non-degradable intrinsically or in association with a protective layer, other than PVC and chosen from the family comprising polyethylene (PE) and in particular linear low-density polyethylene (LLDPE), polyethylene terephthalate (PET), a polyamide (PA), polyethylene vinyl acetate (EVA), ethylene vinyl alcohol (EVOH), styrene ethylene butadiene styrene (SEBS), polyethylene terephthalate glycol (PETG...), polyvinylidene fluoride (PVDF), i.e. suitable for performing the functions making it possible to satisfy the other physical, chemical, biological and operational properties adapted to the relevant purpose and application of the single-use biopharmaceutical device (1),
- the contact layer (16) of the bag (2), of another segment of tube (5) and/or of a connector (4), is made of a material chosen to be identical or analogous to the material of the contact layer (12) of the at least one biopharmaceutical tube (5) ,
- the contact layer (12) of the biopharmaceutical tube (5) is welded to the contact layer (16) of the bag (2), of another tube (5) segment and/or of a connector (4), with substantial physical continuity between the respective contact layers (12, 16) .

2. The single-use biopharmaceutical device (1) for producing, storing, transporting biopharmaceutical product according to claim 1, **characterized in that** a biopharmaceutical bag (2), optionally another segment of biopharmaceutical tube (5) and/or a biopharmaceutical connector (4), welded to at least one biopharmaceutical tube (5), has a multilayer structure identical or analogous to the multilayer structure of the at least one multilayer biopharmaceutical tube (5), namely comprising a contact layer (16) and a functional layer (17) comprising at least one functional elementary layer (17i).

3. The single-use biopharmaceutical device (1) for producing, storing, transporting biopharmaceutical product according to claim 2, **characterized in that** the functional layer (13) of the at least one biopharmaceutical tube (5) is welded to the functional layer (17) of the biopharmaceutical bag (2), optionally of the other segment of biopharmaceutical tube (5) and/or of the biopharmaceutical connector (4).

4. The single-use biopharmaceutical device (1) for producing, storing, transporting biopharmaceutical product according to any one of claims 1 to 3, having at least one of the following characteristics:
- the at least one biopharmaceutical tube (5) is welded to the other component element of the biopharmaceutical device (1) either directly, thereby intrinsically forming the biopharmaceutical connector (4), or indirectly via a biopharmaceutical connector (4);
- it is structurally and functionally associated with the single-use biopharmaceutical device, in a removable or irremovable manner, at least one device for stopping the flow of the biopharmaceutical product in the longitudinal empty space (11) of the at least one biopharmaceutical tube (5) having the form of a clamp suitable for closing the portion of the at least one biopharmaceutical tube (5) at which it is located by flattening the at least one biopharmaceutical tube (5) on itself;
- it constitutes a bag for storing and/or filling and/or handling and/or transporting and/or mixing a biopharmaceutical product or a bioreactor of a biopharmaceutical product.

5. The single-use biopharmaceutical device (1) for producing, storing, transporting biopharmaceutical product according to any one of the preceding claims, **characterized in that** said biopharmaceutical tube (5) consists of the contact layer (12) and the functional layer (13), the functional layer (13) forming the outer face (14b) of the multilayer biopharmaceutical tube (5).

6. The single-use biopharmaceutical device (1) for producing, storing, transporting biopharmaceutical product according to any one of the preceding claims, **characterized in that** the biopharmaceutical tube (5) comprises a single functional elementary layer (13i).

7. The single-use biopharmaceutical device (1) for producing, storing, transporting biopharmaceutical product according to any one of claims 1 to 6, **characterized in that** the biopharmaceutical tube (5) comprises a plurality of elementary functional layers (13i), each secured to the adjacent layer(s).

8. The single-use biopharmaceutical device (1) for producing, storing, transporting biopharmaceutical product according to any one of the preceding claims, **characterized in that** the connection between the outer face (15a) of the contact layer (12) and the inner face (15b) of the functional layer (13) is ensured by manufacture, by co-extrusion or embedding or cladding.

9. The single-use biopharmaceutical device (1) for producing, storing, transporting biopharmaceutical product according to claim 6, **characterized in that** the biopharmaceutical tube (5) includes, associated with a contact layer (12) of LLDPE, a functional elementary layer (13a) of PE.

10. The single-use biopharmaceutical device (1) for producing, storing, transporting biopharmaceutical product according to claim 7, **characterized in that** the biopharmaceutical tube (5) includes one of the following characteristics:
- associated with a contact layer (12) of LLDPE, it includes a functional elementary layer (13a) of EVOH, a functional elementary layer (13b) of PA and a functional elementary layer (13c) of PET that forms the outer face (14b) of the multilayer biopharmaceutical tube (5);
- associated with a contact layer (12) of LLDPE, it includes a functional elementary layer (13a) of EVOH, a functional elementary layer (13b) of PA, two elementary functional layers (13c) of LLDPE and a functional elementary layer (13d) of a biodegradable material that forms the outer face (14b) of the multilayer biopharmaceutical tube (5);
- associated with a contact layer (12) of LLDPE, it includes a functional elementary layer (13a) of a biodegradable material, two elementary functional layers (13b) of LLDPE, a functional elementary layer (13c) of EVOH and a functional elementary layer (13d) of PA that forms the outer face (14b) of the multilayer biopharmaceutical tube (5);
- associated with a contact layer (12) of PE, it includes a functional elementary layer (13a) of EVA, a functional elementary layer (13b) of EVOH, a functional elementary layer (13c) of EVA, and a functional elementary layer (13d) of PE that forms the outer face (14b) of the multilayer biopharmaceutical tube (5);
- associated with a contact layer (12) of LLDPE, it includes a functional elementary layer (13a) of EVOH, a functional elementary layer (13b) of LLDPE and a functional elementary layer (13c) of PE that forms the outer face of the multilayer biopharmaceutical tube (5).

11. The single-use biopharmaceutical device (1) for producing, storing, transporting biopharmaceutical product according to any one of the preceding claims, **characterized in that** the biopharmaceutical tube (5) includes at least one of the following characteristics:
- it has a circular or oblong cross-section;
- it includes a single longitudinal empty space or a plurality of longitudinal empty spaces juxtaposed with leaktight separation.

12. The single-use biopharmaceutical device (1) for producing, storing, transporting biopharmaceutical product according to any one of the preceding claims, **characterized in that** the biopharmaceutical tube (5) is homogeneous from one end to the other of the segment that it forms.

13. The single-use biopharmaceutical device (1) for producing, storing, transporting biopharmaceutical product according to any one of the preceding claims, **characterized in that** the biopharmaceutical tube (5) includes at least at one end of the segment that it forms an uncovered projecting portion constituted by the contact layer, such a segment of multilayer biopharmaceutical tube (5) also performing the function of connector for the single-use biopharmaceutical device (1) for producing, storing, transporting biopharmaceutical product.

14. A method of manufacturing a single-use biopharmaceutical device (1) for producing, storing, transporting biopharmaceutical product according to any one of claims 1 to 13, wherein disposed of at least one biopharmaceutical bag (2), at least one segment of biopharmaceutical tube (5) and, optionally, at least one biopharmaceutical connector (4), and positioning these elements in the configuration of the biopharmaceutical device to be manufactured and welding them together in communicating, continuous, rigid and leaktight manner, so that the contact layer (12) of the at least one biopharmaceutical tube (5) be welded to the contact layer (16) of the biopharmaceutical bag (2), of another segment of biopharmaceutical tube (5) or, optionally, of the biopharmaceutical connector (4), with substantial physical continuity between the respective contact layers (12, 16).
